# EUROPEAN PATENT APPLICATION

(11) **EP 0 724 879 A2**
(43) Date of publication of application: **07.08.1996**
(21) Application number: 96300767.9
(22) Date of filing: 05.02.1996
(51) Int. Cl.: A61K 31/40, A61K 31/445, A61K 31/55

(54) **2-Phenyl-3-azoylbenzothiopenes for inhibiting effects of IL-6**

(30) Priority: 06.02.1995 US 384730
(71) Applicant: ELI LILLY AND COMPANY, Indianapolis, Indiana 46285 (US)
(72) Inventor: Glasebrook, Andrew Lawrence, Zionsville, Indiana 46077 (US); Zuckerman, Steven Harold, Indianapolis, Indiana 46250 (US)
(74) Representative: Hudson, Christopher Mark

(57) **Abstract**

A method of inhibiting the effects of IL-6 comprising administering to a human in need thereof an effective amount of a compound having the formula 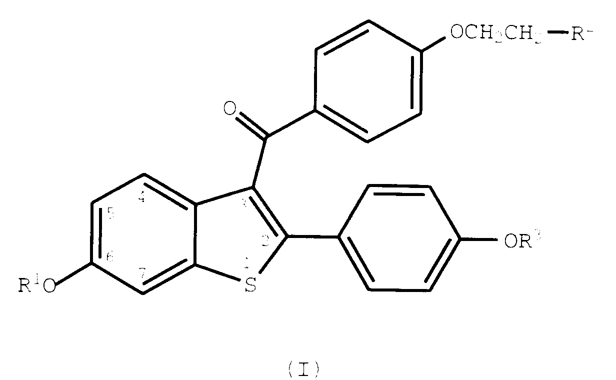
wherein R¹ and R³ are independently hydrogen, -CH₃, 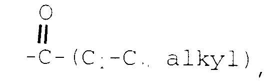
or 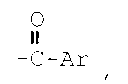
wherein Ar is optionally substituted phenyl;
R² is selected from the group consisting of pyrrolidine, hexamethyleneimino, and piperidino; or a pharmaceutically acceptable salt of solvate thereof.

## Description

Interleukin 6(IL-6) is a multifunctional cytokine produced by various cells. The molecular cloning of the cDNAs encoding B cell stimulatory factor 2(BSF-2), interferon-β2, and 26-kDa protein showed that all these molecules are identical. Furthermore, hybridoma/plasmacytoma growth factor (HPGF) and hepatocyte stimulating factor (HSF) were also found to be identical to the molecule and, therefore, this molecule has been called IL-6. Subsequent studies demonstrated that IL-6 acted not only on B cells but also on hematopoietic stem cells and hepatocytes and induced hematopoiesis as well as acute phase reactions. It has been also shown to act on T cells, nerve cells, keratinocytes, renal mesangial cells, megakaryocytes, and myeloma/plasmacytoma cells. Since antibody production, hematopoiesis, and acute phase reactions are three major responses against infection, inflammation, and tissue injuries, IL-6 may have a central role in host defense mechanisms. On the other hand, the deregulation of IL-6 gene expression was shown to be involved in the pathogenesis of polyclonal and monoclonal B cell abnormalities, such as rheumatoid arthritis and multiple myelomas.

Interleukin 6 was originally identified as a T cell derived lymphokine that induces final maturation of B cells into antibody-producing cells. Recombinant human IL-6 acts on B cells activated with *Staphylococcus aureus* Cowan I or pokeweed mitogen (PWM) to induce IgM, IgG, and IgA production, but not on resting B cells. Anti-IL-6 antibody was found to inhibit PWM-induced Ig production, indicating that IL-6 is one of the essential factors in PWM-induced Ig production. Furthermore, IL-6 was shown to augment the primary and secondary anti-SRBC antibody production in mice *in vivo.* Also IL-6 could enhance IgA synthesis in murine Peyer's patch B cells which were already committed to IgA production. Murine IL-6 was also shown to act on murine B cells activated with anti-Ig or dextran sulfate; IL-6 and IL-1 synergistically stimulate the growth and differentiation of these murine B cells. Interleukin 6 could also induce the growth and differentiation of T cells and promoted the growth of mitogen-stimulated thymocytes and peripheral T cells. It was also shown to induce the differentiation of cytotoxic T cells in the presence of IL-2 from murine as well as human thymocytes and splenic T cells.

By shortening the Go period of the stem cells, IL-6 and IL-3 synergistically induce the colony formation of multipotent hematopoietic progenitors. This synergic effect of IL-3 and IL-6 was also observed in serum-free culture conditions, suggesting that IL-6 may enhance the sensitivity of multipotent stem cells to IL-3. When bone marrow cells were transplanted to lethally irradiated recipients, the survival rate at day 30 was only 20%. However, when these cells were precultured with IL-6 and IL-3 before transplantation, the survival rate was raised to 90%.

Furthermore, IL-6 induced the maturation of megakaryocytes *in vitro* and *in vivo.* IL-6 promoted marked increments in size and acetylcholinesterase activity, a marker enzyme of this lineage. IL-6 also induced a significant shift toward higher ploidy classes.

The acute phase response is a systemic reaction against inflammation, infection, or tissue injury, which is characterized by leukocytosis, fever, increased vascular permeability, alteration in plasma metal and steroid concentration, along with increased levels of acute phase proteins. The production of acute phase proteins by hepatocytes is regulated by several soluble factors, such as IL-1, TNF, and HSF. Among these factors, only HSF could induce the full acute phase proteins. It was demonstrated that recombinant IL-6 can function as HSF. It can induce various acute phase proteins, such as fibrinogen, α-1-antichymotrypsin, α-1-acid glycoprotein, and haptoglobin, in a human hepatoma cell line. In addition, IL-6 induces serum amyloid A, C-reactive protein, and α-1-antitrypsin in human primary hepatocytes. In the rat, IL-6 induces fibrinogen, cysteine proteinase inhibitor, and α2 macroglobulin. Serum albumin was negatively regulated by IL-6.

Interleukin 6 mRNA was induced by IL-1 stimulated glioblastoma cells or astrocytoma cells, suggesting that IL-6 may have certain effects on neural cells. The rat pheochromocytoma cell line, PC12, is a typical neural differentiation model. Nerve growth factor (NGF) induces chemical, ultrastructural, and morphological changes in the PC12 cell. Also IL-6 was found to induce the typical differentiation of this cell into neural cells and found to be produced by virus-infected microglial cells and astrocytes. It also induced the secretion of NGF by astrocytes. In CNS IL-6 production may be involved in repair mechanisms in the course of viral infection.

The involvement of IL-6 in disease was first suggested in cardiac myxoma. The patients frequently show symptoms related to polyclonal plasmacytosis, such as hypergammaglobulinemia and the presence of various autoantibodies and an increase in acute phase proteins. These symptoms disappear upon the resection of the tumor, suggesting that myxoma-derived factors may induce these phenomena. It was found that myxoma cells express a large amount of IL-6. Abnormal IL-6 production was also observed in patients with Castleman's disease. In these patients, activated B cells in the germinal center of hyperplactic lymph nodes were found to produce IL-6. After the resection of these lymph nodes, clinical improvement and a decrease in serum IL-6 levels were observed. This evidence suggests that deregulated IL-6 production may induce polyclonal B cell activation and increases in acute phase proteins. This possibility was also suggested in rheumatoid arthritis (RA). High levels of IL-6 were detected in synovial fluid from the joints of patients with active RA. Interleukin 6 is a potent growth factor for murine hybridomas/plasmacytomas, suggesting a possible involvement of IL-6 in the generation of plasmacytomas/myelomas. Furthermore, a study with human myeloma cells isolated from patients with multiple myelomas demonstrated that IL-6 is an autocrine growth factor for human myeloma cells. All the evidence suggests that deregulated gene expression of IL-6 may be involved in the polyclonal B cell activation and generation of plasma cell neoplasia.

Mesangial proliferative glomerulonephritis (PGN) is histologically characterized by proliferation of mesangial cells (MC), suggesting the involvement of the growth factor for MC in the pathogenesis of this disease. It was shown that IL-6 is an autocrine growth factor for MC. It could be detected in urine samples from patients with PGN. Furthermore, a close relationship was observed between the level of urine IL-6 and the progression of PGN. These results suggest that deregulated IL-6 production in MC is involved in the pathogenesis of PGN.

Other diseases in which excess IL-6 is implicated include rheumatoid arthritis, multiple myeloma, lupus, Hashmito's autoimmune thyroiditis and autoimmune hemolytic anemia.

This invention provides methods for inhibiting the effects of IL-6 comprising administering to a human in need thereof an effective amount of a compound of formula I 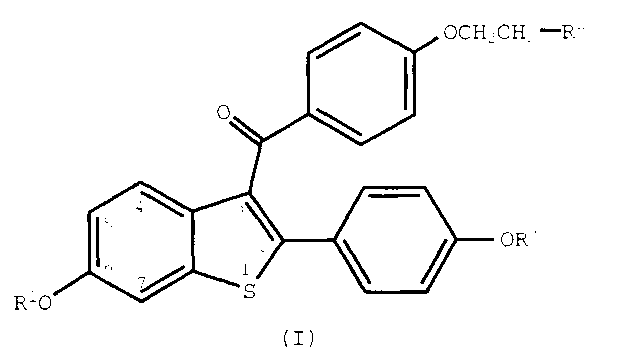 wherein
R¹ and R³ are independently hydrogen, -CH₃, 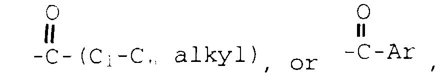 wherein Ar is optionally substituted phenyl;
R² is selected from the group consisting of pyrrolidino, hexamethyleneimino, and piperidino; and pharmaceutically acceptable salts and solvates thereof.

The current invention concerns the discovery that a select group of 2-phenyl-3-aroylbenzothiophenes (benzothiophenes), those of formula I, are useful for inhibiting the effects of IL-6. Also, the compounds are useful for inhibiting the effects of leukemia inhibitor factor (LIF). Lastly, the compounds are useful for inhibiting receptor systems using the gp130 transmembrane protein for binding/signal transduction. Such receptor systems include IL-6, LIF, onconstatin M, ciliary neurotrophic factor, and IL-11.

The methods of use provided by this invention are practiced by administering to a human in need thereof a dose of a compound of formula I or a pharmaceutically acceptable salt or solvate thereof, that is effective to inhibiting the effects of IL-6.

The term "inhibit" includes its generally accepted meaning which includes prohibiting, preventing, restraining, and slowing, stopping or reversing. As such, the present method includes both medical therapeutic and/or prophylactic administration, as appropriate. While not wishing to be bound by theory, it is believed that the compounds of formula I may inhibit secretion and/orutilization of IL-6, and therefore be useful for disorders associated with an excess of IL-6. Further, the comounds appear to inhibit receptor systems utilizing the gp130 transmembrane protein for binding/signal transduction.

Raloxifene, a compound of this invention wherein it is the hydrochloride salt of a compound of formula 1, R¹ and R³ are hydrogen and R² is 1-piperidinyl, is a nuclear regulatory molecule. Raloxifene has been shown to bind to the estrogen receptor and was originally thought to be a molecule whose function and pharmacology was that of an anti-estrogen in that it blocked the ability of estrogen to activate uterine tissue and estrogen dependent breast cancers. Indeed, raloxifene does block the action of estrogen in some cells; however in other cell types, raloxifene activates the same genes as estrogen does and displays the same pharmacology, e.g., anti-osteoporosis, hyperlipidemia. As a result, raloxifene has been referred to as an anti-estrogen with mixed agonist-antagonist properties. The unique profile which raloxifene displays and differs from that of estrogen is now thought to be due to the unique activation and/or suppression of various gene functions by the raloxifene-estrogen receptor complex as opposed to the activation and/or suppression of genes by the estrogen-estrogen receptor complex. Therefore, although raloxifene and estrogen utilize and compete for the same receptor, the pharmacological outcome from gene regulation of the two is not easily predicted and is unique to each.

Generally, the compound is formulated with common excipients, diluents or carriers, and compressed into tablets, or formulated as elixirs or solutions for convenient oral administration, or administered by the intramuscular or intravenous routes. The compounds can be administered transdermally, and may be formulated as sustained release dosage forms and the like.

The compounds used in the methods of the current invention can be made according to established procedures, such as those detailed in U.S. Patent Nos. 4,133,814, 4,418,068, and 4,380,635 all of which are incorporated by reference herein. In general, the process starts with a benzo[b]thiophene having a 6-hydroxyl group and a 2-(4-hydroxyphenyl) group. The starting compound is protected, acylated, and deprotected to form the formula I compounds. Examples of the preparation of such compounds are provided in the U.S. patents discussed above. Optionally substituted phenyl includes phenyl and phenyl substituted once or twice with C₁-C₆ alkyl, C₁-C₄ alkoxy, hydroxy, nitro, chloro, fluoro, or tri(chloro or fluoro)methyl.

The compounds used in the methods of this invention form pharmaceutically acceptable acid and base addition salts with a wide variety of organic and inorganic acids and bases and include the physiologically acceptable salts which are often used in pharmaceutical chemistry. Such salts are also part of this invention. Typical inorganic acids used to form such salts include hydrochloric, hydrobromic, hydroiodic, nitric, sulfuric, phosphoric, hypophosphoric and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, teraphthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzenesulfonate, p-bromophenylsulfonate, chlorobenzenesulfonate, ethanesulfonate, 2-hydroxyethanesulfonate, methanesulfonate, naphthalene-1-sulfonate, naphthalene-2-sulfonate, p-toluenesulfonate, xylenesulfonate, tartarate, and the like. A preferred salt is the hydrochloride salt.

The pharmaceutically acceptable acid addition salts are typically formed by reacting a compound of formula I with an equimolar or excess amount of acid. The reactants are generally combined in a mutual solvent such as diethyl ether or benzene. The salt normally precipitates out of solution within about one hour to 10 days and can be isolated by filtration or the solvent can be stripped off by conventional means.

Bases commonly used for formation of salts include ammonium hydroxide and alkali and alkaline earth metal hydroxides, carbonates, as well as aliphatic and primary, secondary and tertiary amines, aliphatic diamines. Bases especially useful in the preparation of addition salts include ammonium hydroxide, potassium carbonate, methylamine, diethylamine, ethylene diamine and cyclohexylamine.

The pharmaceutically acceptable salts generally have enhanced solubility characteristics compared to the compound from which they are derived, and thus are often more amenable to formulation as liquids or emulsions.

Pharmaceutical formulations can be prepared by procedures known in the art. For example, the compounds can be formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

The compounds can also be formulated as elixirs or solutions for convenient oral administration or as solutions appropriate for parenteral administration, for instance by intramuscular, subcutaneous or intravenous routes. Additionally, the compounds are well suited to formulation as sustained release dosage forms and the like. The formulations can be so constituted that they release the active ingredient only or preferably in a particular part of the intestinal tract, possibly over a period of time. The coatings, envelopes, and protective matrices may be made, for example, from polymeric substances or waxes.

The regimen and particular dosage of a compound of formula I required to inhibit the effects of IL-6, LIF, or receptor systems which use the gp130 transmembrane, according to this invention will depend upon the severity of the condition, the route of administration, and related factors that will be decided by the attending physician. Generally, accepted and effective daily doses will be from about 0.1 to about 1000 mg/day, and more typically from about 50 to about 200 mg/day. Such dosages will be administered to a subject in need thereof from once to about three times each day, or more often as needed, and for a time sufficient to effectively inhibit the effects of IL-6.

It is usually preferred to administer a compound of formula I in the form of an acid addition salt, as is customary in the administration of pharmaceuticals bearing a basic group, such as the piperidino ring. It is also advantageous to administer such a compound by the oral route. For such purposes the following oral dosage forms are available.

### Formulations

In the formulations which follow, "Active ingredient" means a compound of formula I.

### Formulation 1: Gelatin Capsules

Hard gelatin capsules are prepared using the following:

| Ingredient | Quantity (mg/capsule) |
|---|---|
| Active ingredient | 0.1 - 1000 |
| Starch, NF | 0 - 650 |
| Starch flowable powder | 0 - 650 |
| Silicone fluid 350 centistokes | 0 - 15 |

The ingredients are blended, passed through a No. 45 mesh U.S. sieve, and filled into hard gelatin capsules.

Examples of specific capsule formulations of raloxifene that have been made include those shown below:

### Formulation 2: Raloxifene capsule

| Ingredient | Quantity (mg/capsule) |
|---|---|
| Raloxifene | 1 |
| Starch, NF | 112 |
| Starch flowable powder | 225.3 |
| Silicone fluid 350 centistokes | 1.7 |

### Formulation 3: Raloxifene capsule

| Ingredient | Quantity (mg/capsule) |
|---|---|
| Raloxifene | 5 |
| Starch, NF | 108 |
| Starch flowable powder | 225.3 |
| Silicone fluid 350 centistokes | 1.7 |

### Formulation 4: Raloxifene capsule

| Ingredient | Quantity (mg/capsule) |
|---|---|
| Raloxifene | 10 |
| Starch, NF | 103 |
| Starch flowable powder | 225.3 |
| Silicone fluid 350 centistokes | 1.7 |

### Formulation 5: Raloxifene capsule

| Ingredient | Quantity (mg/capsule) |
|---|---|
| Raloxifene | 50 |
| Starch, NF | 150 |
| Starch flowable powder | 397 |
| Silicone fluid 350 centistokes | 3.0 |

The specific formulations above may be changed in compliance with the reasonable variations provided.

A tablet formulation is prepared using the ingredients below:

### Formulation 6: Tablets

| Ingredient | Quantity (mg/tablet) |
|---|---|
| Active ingredient | 0.1 - 1000 |
| Cellulose, microcrystalline | 0 - 650 |
| Silicon dioxide, fumed | 0 - 650 |
| Stearate acid | 0 - 15 |

The components are blended and compressed to form tablets.

Alternatively, tablets each containing 0.1 - 1000 mg of active ingredient are made up as follows:

### Formulation 7: Tablets

| Ingredient | Quantity (mg/tablet) |
|---|---|
| Active ingredient | 0.1 - 1000 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60° C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.1 - 1000 mg of medicament per 5 mL dose are made as follows:

### Formulation 8: Suspensions

| Ingredient | Quantity (mg/5 ml) |
|---|---|
| Active ingredient | 0.1 - 1000 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The medicament is passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.

### Assay 1

Six month old, virgin SD rats are OVX or OVX/hypophysectomized (HYPOX) and treated with vehicle (20% cyclodextrin, PO), raloxifene (0.1 to 10 mg/kg/day, PO) or ethynyl estradiol EE2; 0.001 to 0.1 mg/kg/day, PO). A sham, vehicle treated control is also included. Serum and/or long bones are collected after a 35 day or shorter treatment period. Serum cytokine determinations are performed by bioassay.

OVX results in a marked decrease in bone mass after a 35 day treatment which is accompanied by a marked and consistent increase in serum IL-6 levels, (Table 1) Interestingly, both raloxifene and EE2 significantly attenuate both the OVX-induced osteopenia and the rise in serum IL-6 levels. In animals treated for as few as four days post-OVX, a significant reduction is produced by EE2 and raloxifene treatment. In contrast, no increase in serum IL-6 is observed in OVX/HYOX rats which also did not respond to either EE2 or raloxifene in terms of bone density. No consistent changes in other serum cytokines is detected.

**Table 1**

| | Serum IL-6(ng/ml) | BMD % (protection) |
|---|---|---|
| Sham | 0.42 ± 0.13 | 100 ± 4.65 |
| OVX | 7.18 ± 2.0 | 0.00 ± 10.24 |
| Ralox 10 mg/kg | 1.23 ± 0.45 | 82.09 ± 7.02 |
| Ralox 1 mg/kg | 1.55 ± 0.48 | 82.50 ± 17.17 |
| Ralox 0.1 mg.kg | 4.32 ± 1.65 | 63.88 ± 19.8 |
| Ethy 100 µg/kg | 0.20 ± 0.10 | 76.00 ± 18.3 |
| Estradiol 10 µg/kg | 1.84 ± 0.60 | 57.25 ± 16.07 |
| Estradiol 1 µg/kg | 3.42 ± 2.23 | 5.28 ± 11.83 |
| 17-β-E2 100 µg/kg | 0.07 ± 0.10 | 106.4 ± 11.8 |

### Assay 2

Ten week old male BALB/c mice are treated with vehicle (20% cyclodextrin, PO) or raloxifene (1 mg/kg, PO) for 3 days. On day 4, mice are treated with vehicle (saline, IP), LA-15.1 anti-IL-1 receptor monoclonal antibody (mAb) (250 ug, IP) or human recombinant IL-1b (3 ug, SC). After 2 hours, serum was collected by orbital sinus puncture and IL-6 levels quantitated by ELISA.

Injection of IL-1 into cyclodextrin treated mice induces a significant increase in serum IL-6 (193 ± 29 ng/ml) versus saline injected cyclodextrin treated mice (<1 ng/ml) (Table 2). Interestingly, raloxifene significantly attenuates the IL-1-induced rise in serum IL-6 levels (90 ± 21 ng/ml). The anti-IL-1 receptor mAb LA-15.1 completely attenuates the IL-1-induced rise in serum IL-6 levels.

**Table 2**

| | IL-6 (ng/ml) |
|---|---|
| CDX/Sal/Sal | <0.5 |
| CDX/Sal/IL-1 | 193 ± 2.9 |
| 139478/Sal/IL-1 | 90 ± 2.1 |
| CDX/15.6 MAb/IL-1 | <0.5 |

### Assay 3

The following mouse cell lines were used for cytokine bioassays: CTLL.6 cytolytic T cell - IL-2, IL-4; 11.6 helper T cell - IL-4; T1165.17 plasmacytoma - IL-1, IL-6; B9 hybridoma - IL-6, LIF (leukemia inhibitory factor). Approximately 5,000 cells are seeded per duplicate well in 96-well flat-bottom microtiter plates in standard tissue culture medium (i.e. Iscove's Modified Dulbecco's Medium or Dulbecco's Modified Eagle's Medium supplemented with 25 mM Hepes, 2 mM L-glutamine, 50 µM 2-mercaptoethanol, 50 units/ml penicillin, 50 µg/ml streptomycin sulfate, and fetal bovine serum) containing vehicle (DMSO), raloxifene (0.01 to 1 µM) or 17-b-estradiol (0.01 to 1 µM) in the absence or presence of the indicated recombinant cytokine. Microtiter plates are incubated at 37°C in a humidified 10% CO₂ incubator for 24 to 72 hours. At the end of that time, either 1 µCi 3H-thymidine or 100 µg of MTT (3-[4,5-dimethylthiazol-2-yl]-2,5-diphenyltetrazolium bromide) is added per well followed by an additional 4 to 6 hours of incubation. Microcultures pulsed using tritiated thymidine are harvested onto filter pads and counted by liquid scintillation. Microcultures pulsed using MTT receive 100 µl of 10% SDS / 0.01N HCl per well followed by overnight incubation in the dark at room temperature.
Optical density readings are taken at 570 nm with a reference wavelength of 690 nm to quantitate cell proliferation.

The results comparing raloxifene to estrogen for inhibition of cytokine dependent proliferation are shown in Table 3. Data are expressed as an IC₅₀ value for 50% inhibition of cytokine dependent cell proliferation. Interestingly, raloxifene significantly attenuates IL-6 and LIF stimulated cell proliferation (IL-6 IC₅₀ = 719 nM; LIF IC₅₀ = 603 nM), but has no effect on IL-2 and IL-4 stimulated proliferation (IC₅₀ > 1 µM). In contrast, 17-b-estradiol doesn't significantly inhibit cell proliferation by any cytokine examined (IC₅₀ = >1 µM). These results suggest that raloxifene inhibits cell proliferation stimulated by cytokine binding to surface receptors utilizing the gp130 transmembrane protein for binding/signal transduction. Such receptor systems include IL-6, LIF, oncostatin M, ciliary neurotrophic factor, and IL-11.

**Table 3**

| IC₅₀ Values | | |
|---|---|---|
| Cytokine | 17-B-E2 | Raloxifene |
| IL-6 | >1µM (n=2) | 719 (n=2) |
| LIF | >1µM (n=6) | 603 ± 212 nM (n=6) |
| IL-2 | >1µM (n=1) | >1µm (n=1) |
| IL-4 | >1µM (n=3) | >1µm (n=3) |

### Assay 4

Female non ovex mice are treated with raloxifene or analogs and after 3-4 days of dosing these mice are stimulated with a dose of lipopolysaccharide between 20-200 ug per mouse. At 3 hours post stimulus, animals are bled and sera IL-6 determinations are performed. The 3 hour timepoint based on preliminary experiments represents the peak in Il-6 detection.

In a separate series of experiments while estrogen analogs were able to reduce IL-6 levels in LPS stimulated mice, the combination of estrogen plus raloxifene analog resulted in a synergistic reductio in IL-6, greater than that ovserved for either agent alone.

Therefore, it is envisioned tha the most effective way of reducing IL-6 levels in vivo will be to combine a low dose estrogen with raloxifene.

Final assay would involve either exvivo or in vitro experiments in which macrophages are exposed to raloxifene or analogs in the presence or absence of estrogen and supernatant IL-6 levels would be measured following an in vitro LPS stimulus.

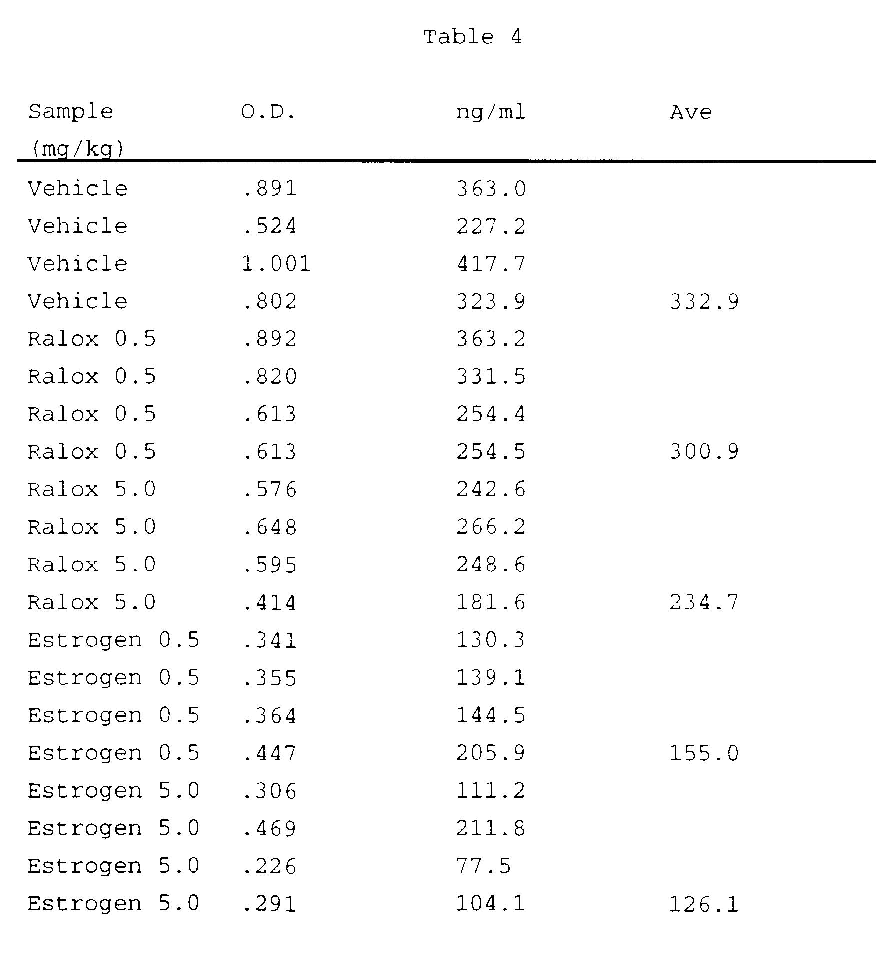

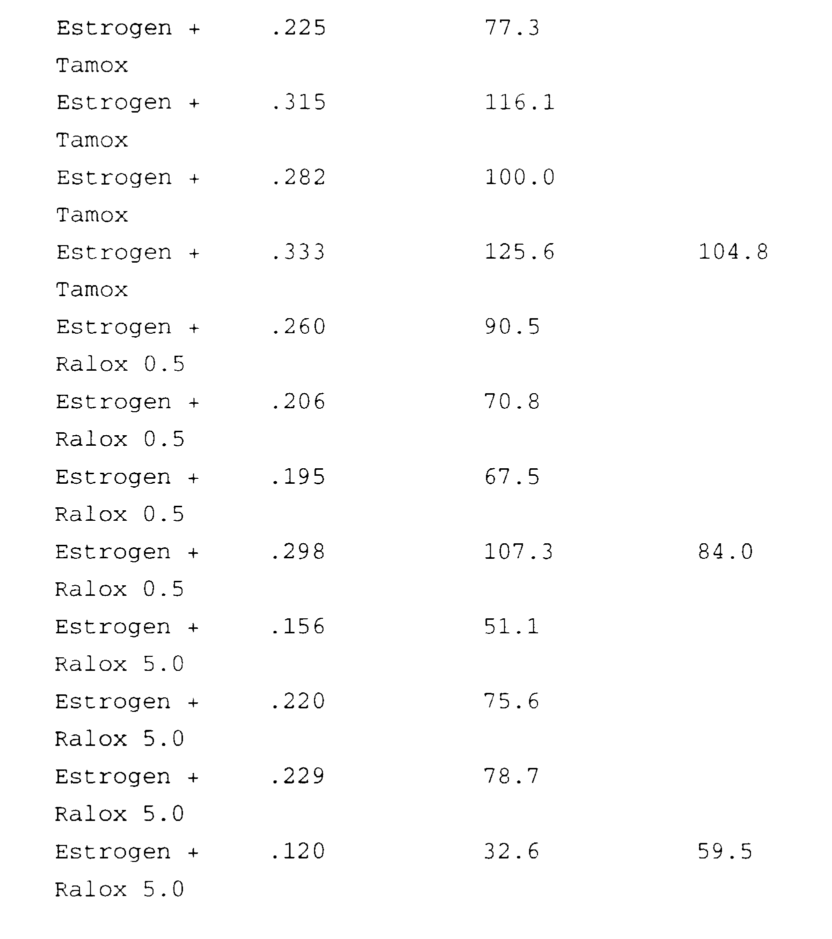

## Claims

1. The use of a compound having the formula 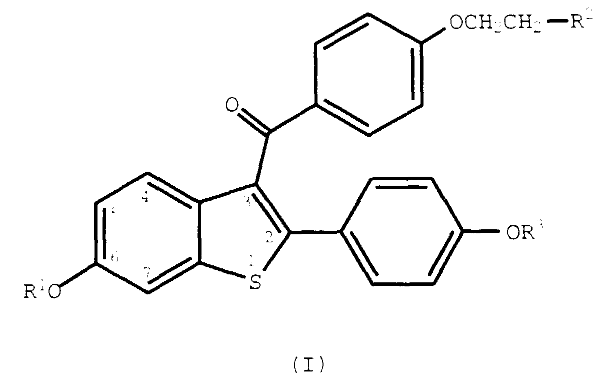 wherein
R¹ and R³ are independently hydrogen, -CH₃, 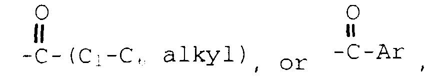 wherein Ar is optionally substituted phenyl;
R² is selected from the group consisting of pyrrolidine, hexamethyleneimino, and piperidino; or a pharmaceutically acceptable salt of solvate thereof, in the preparation of a medicament useful for inhibiting the effects of IL-6.

2. The use of Claim 1 wherein said compound is the hydrochloride salt thereof.

3. The use of Claim 1 wherein said compound is 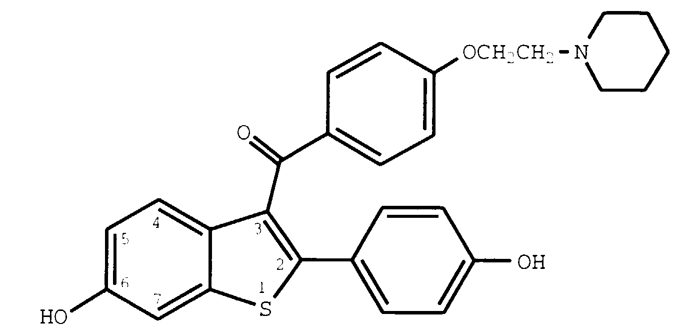 or its hydrochloride salt.

4. The use of a compound having the formula I 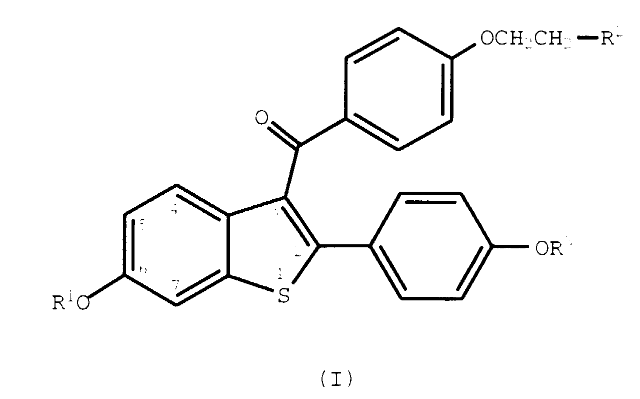 wherein
R¹ and R³ are independently hydrogen, 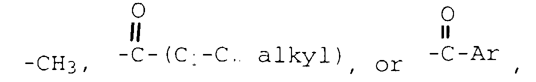 wherein Ar is optionally substituted phenyl;
R² is selected from the group consisting of pyrrolidine, hexamethyleneimino, and piperidino; or a pharmaceutically acceptable salt of solvate thereof, in the preparation of a medicament useful for inhibiting the effects of LIF.

5. A use of a compound of formula I 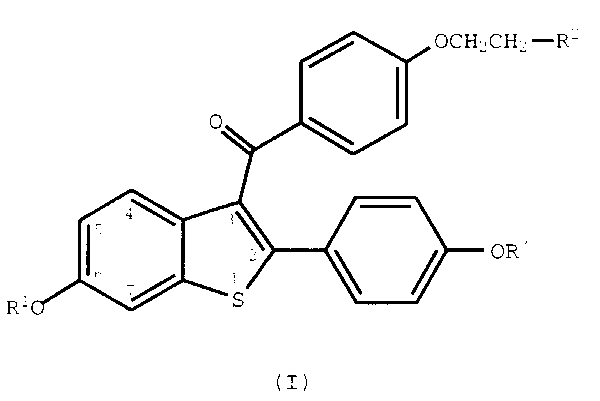 wherein
R¹ and R³ are independently hydrogen, 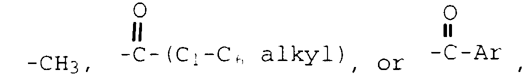 wherein Ar is optionally substituted phenyl;
R² is selected from the group consisting of pyrrolidine, hexamethyleneimino, and piperidino; or a pharmaceutically acceptable salt of solvate thereof, in the preparation of a medicament useful for inhibiting a surface receptor system utilizing the gp130 transmembrane receptor.
